# EUROPÄISCHE PATENTSCHRIFT

(11) **EP 1 714 608 B1**
(45) Veröffentlichungstag und Bekanntmachung des Hinweises auf die Patenterteilung: **19.03.2008**
(21) Anmeldenummer: 06007249.3
(22) Anmeldetag: 06.04.2006
(51) Int. Cl.: A61B 1/12

(54) **Endoskop mit einem Ablenklelement für Spülmedien**
Endoscope with deflection element for cleaning fluids or air
Endoscope avec un élément pour déflectionner un liquide de nettoyage ou air

(30) Priorität: 20.04.2005 DE 102005019142
(43) Veröffentlichungstag der Anmeldung: 25.10.2006
(73) Patentinhaber: Karl Storz GmbH & Co. KG, 78532 Tuttlingen (DE)
(72) Erfinder: Jerjomin, Vitali, 12618 Tallinn (IS)
(74) Vertreter: Weller, Wolfgang

(56) Entgegenhaltungen:
- WO-A-20/06014814
- US-A- 4 881 810
- US-A- 5 746 695
- US-A- 5 772 579
- US-B1- 6 409 657
- PATENT ABSTRACTS OF JAPAN Bd. 018, Nr. 219 (C-1192), 20. April 1994 (1994-04-20) & JP 06 014865 A (ASAHI OPTICAL CO LTD), 25. Januar 1994 (1994-01-25)

## Beschreibung

Die Erfindung betrifft ein Endoskop, insbesondere ein flexibles Videoendoskop, mit einem Schaft, der distal in einem Endoskopkopf endet, der zumindest einen Lichteinlass und zumindest einen Kanal zum Durchführen eines Spülmediums aufweist, sowie mit einem Ablenkelement, das im Abstand vor einer Mündung des Kanals angeordnet ist, um das austretende Spülmedium zum Lichteinlass zu lenken, wobei das Ablenkelement an einem Bruchstück des Körpers des Endoskopkopfes angeordnet ist, das vom Körper abnehmbar ist, und dass das Bruchstück lagedefiniert und unverrückbar am Körper aufgenommen ist und über eine Halterung am Körper befestigbar ist, wie im Oberbegriff des Anspruchs 1 erwähnt.

Ein derartiges Endoskop ist beispielsweise aus der US 6,409,657 B1 bekannt.

Derartige Endoskope werden insbesondere dazu eingesetzt, um körperinnere Hohlorgane zu betrachten. Bei flexiblen Endoskopen können beispielsweise die Luftröhre, die Speiseröhre, der Magen oder der Darm untersucht werden.

Am distalen Ende ist ein Lichteinlass vorgesehen, über den das Licht des zu beobachtenden Bildes eingeführt und dann bis zum proximalen Ende geleitet wird. Bei starren Endoskopen wird dies über ein Stablinsensystem bewerkstelligt, bei modernen flexiblen Endoskopen befindet sich im distalen Endoskopkopf ein lichtempfindlicher Chip, der die eintretenden Lichtsignale in ein elektrisches Signal umwandelt und zum proximalen Kopfstück leitet. Dort wird das elektrische Signal wieder in ein Bildsignal umgesetzt und beispielsweise auf einem Monitor dargestellt.

Beim praktischen Einsatz wurde nun festgestellt, dass der Licht- bzw. Bildeinlass verschmutzt wird und es notwendig ist, diesen von Verschmutzungen zu befreien.

Dazu ist vorgesehen, durch den Schaft des Endoskops einen Kanal zum Durchführen eines Spülmediums, sei es eines gasförmigen oder eines flüssigen Mediums, vorzusehen. Da dieser Kanal zwangsläufig neben dem Optikkanal verlaufen muss, müssen entsprechende Maßnahmen vorgesehen sein, um das Spülmedium seitlich abzulenken und über den Lichteinlass zu leiten, um ihn dadurch zu reinigen. Dazu wurden nun zahlreiche konstruktive Vorschläge gemacht, wie man diesen Spülstrahl umlenken kann.

Die eingangs erwähnte US 6,409,657 betrifft eine Vorrichtung zum Reinigen eines Bildfensters von Endoskopen. Diese Vorrichtung ist an einer elastischen Kappe angeordnet. In der elastischen Kappe ist ebenfalls eine Nut ausgebildet, mittels der die Kappe mit der Vorrichtung zum Reinigen des Bildfensters an einem Endoskopkopf lagedefiniert befestigt wird. Die Kappe ist derart ausgebildet, dass sie von dem Endoskopkopf abnehmbar ist. In dem Endoskop sind zwei Kanäle vorhanden, wobei ein erster Kanal zum Zuführen eines flüssigen Mediums, z.B. Spülwasser dient und ein zweiter Kanal zum Zuführen eines gasförmigen Mediums, z.B. Luft dient. Die Vorrichtung zum Reinigen des Bildfensters ist derart an der Kappe angeordnet, dass, wenn die Kappe an dem Endoskopkopf befestigt ist, sie etwas im Abstand vor der Mündung der Kanäle liegt, aus denen das Spülmedium austritt. Das austretende Spülmedium trifft dann auf die Vorrichtung, wird von dieser aus einer axialen in eine radiale Richtung seitlich umgeleitet, und der Spülstrahl wird über das an dem Endoskopkopf angeordnete Bildfenster geführt, um dieses dadurch freizuspülen oder freizublasen.

Bei der US 4,436,087 wird das Umlenken des Spülmediums, um den Lichteinlass zu reinigen, dadurch bewerkstelligt, dass auf das distale Ende des Endoskopkopfes eine Kappe aufgesetzt, meistens aufgeschraubt, wird, von deren Innenseite ein Ablenkelement radial nach innen vorspringt.

Dabei ist das Ablenkelement so ausgestaltet, dass es etwas im Abstand vor der Mündung des Kanals zum Liegen kommt, aus dem das Spülmedium austritt. Das austretende Spülmedium trifft dann auf das Ablenkelement, wird von diesem aus der axialen in eine radiale Richtung seitlich umgeleitet, und der Spülstrahl wird über den Lichteinlass geführt, um diesen dadurch freizuspülen oder freizublasen.

Im praktischen Einsatz haben sich bestimmte Nachteile dieser Konstruktion gezeigt.

Der innere Hohlraum der Kappe zeigte sich als eine Art Schmutzfänger und fördert eher die Verschmutzung des Lichteinlasses als bei einem Endoskop ohne eine solche Kappe.

Zum Reinigen eines Spülkanals, beispielsweise indem durch diesen eine Reinigungsbürste in axialer Richtung hin durchgeführt wird, muss die Kappe abgenommen werden, da ansonsten die Bürste gegen das Ablenkelement stoßen würde. Bedenkt man, dass Endoskope einen Durchmesser im Bereich von einigen Millimetern aufweisen, leuchtet ein, dass es sich hier um relativ kleine und empfindliche Bauelemente handelt.

Ein weiterer Nachteil besteht darin, dass die abschließend aufgesetzte Abschlusskappe mit dem Ablenkelement sehr exakt positioniert werden muss, damit auch das gewünschte Ablenkergebnis erzielt werden kann.

Es ist üblich, dass durch den Schaft eines Endoskops mehrere Kanäle geführt werden, also nicht nur der Kanal für die Optik sowie ein Spülkanal, sondern meist zusätzlich noch ein oder mehrere Kanäle zum Führen des Beleuchtungslichtes sowie oftmals noch ein Instrumentenkanal zum Führen eines Instrumentes durch den Schaft hindurch.

Damit das gewünschte Freispülen oder Freiblasen des Lichteinlasses erzielt werden kann, muss dann das Ablenkelement in einer ganz bestimmten Position über der Mündung des Spülkanals zum Liegen kommen, damit der Strahl auch tatsächlich über den Lichteinlass geführt wird und nicht möglicherweise über den Instrumentenkanal. Gleichzeitig muss das Ablenkelement so klein sein, dass es weder den Lichteintritt in den Lichteinlass stört, noch das Beleuchtungslicht abschirmt, oder gar den Instrumentenkanal blockiert. D.h. das Ablenkelement springt von einer inneren Umfangsseite der Kappe in einem relativ kleinen Bereich vor.

Wird nun die Kappe zu wenig oder zu weit aufgedreht, kommt das Ablenkelement nicht in seiner exakt vorbestimmten Position zum Liegen, und das erwünschte Spülergebnis wird nicht erzielt.

Aus der US 5,746,695 ist ein Endoskop der zuvor genannten Art bekannt, bei dem in der Abschlusskappe ein Ablenkelement integriert ist, um das austretende Spülmedium seitlich zum Lichteinlass zu lenken. Im Gegensatz zu der zuvor erwähnten Konstruktion mit einer aufschraubbaren Kappe ist die Kappe so konturiert, dass sie nur in einer ganz bestimmten Stellung auf das distale Ende des Endoskops aufgesetzt werden kann. Dazu sind sowohl an dem distalen Ende des Endoskopkopfes als auch an der Kappe entsprechende komplementäre Vorsprünge bzw. Ausnehmungen vorgesehen, die die lagegerechte Positionierung der Endkappe mit dem Ablenkelement sicherstellen.

Nachteilig ist dabei, dass sowohl die distal endseitige Konstruktion des Endoskopkopfes als auch die entsprechende Seite der Kappe sehr kompliziert geformt sind, was einen sehr aufwändigen Bearbeitungsvorgang notwendig macht. Die zahlreichen Aussparungen und Hinterschneidungen stellen Bakteriennischen dar und erfordern eine sehr aufwändige Reinigung bzw. Sterilisierung.

Es ist daher Aufgabe der vorliegenden Erfindung, einfache konstruktive Maßnahmen vorzusehen, die eine exakte Positionierung des Ablenkelements gewährleisten, um auch nach mehreren Zerleg-, Reinigungs- und Zusammenbauvorgängen einen exakt ausgerichteten Spülstrahl zu erzielen.

Erfindungsgemäß wird die Aufgabe dadurch gelöst, dass die Halterung als Kappe ausgebildet ist, die auf den Zusammenbau aus Bruchstück und Endoskopkopf aufsetzbar ist.

Unter einem Bruchstück im Sinne der vorliegenden Erfindung wird ein Stück des meist etwa zylinderförmigen Körpers des Endoskopkopfes verstanden, das von diesem abgenommen werden kann. Man kann sich dies so vorstellen, dass man von einer aus Backsteinen zusammengesetzten Mauer einen Backstein als Bruchstück herausnimmt.

An diesem Bruchstück ist nun das eigentliche Ablenkelement angeordnet. Durch die Ausgestaltung als Bruchstück ist dieses exakt passend in die entsprechende Lücke des Körpers des Endoskopkopfes einzusetzen. Dadurch erfolgt dann zwangsweise eine exakte Ausrichtung des vom Bruchstück vorstehenden Ablenkelements, so dass dies jedes Mal, nachdem das Bruchstück zum Reinigen des Spülkanals abgenommen und wieder angesetzt wurde, in einer exakten Ausrichtung steht. Das Ansetzen des Bruchstückes an den Körper des Endoskopkopfes ist nur in einer einzigen exakt ausgerichteten Stellung durchzuführen und hängt somit nicht mehr von der Geschicklichkeit oder von der Aufmerksamkeit der Person ab, die den Endoskopkopf nach einem Reinigungsvorgang montiert. Eine Fehlposition durch ein zu geringes oder ein zu starkes Überdrehen einer Kappe, an der das Ablenkelement vorhanden ist, ist somit nunmehr systemimmanent ausgeschlossen.

Man kann somit einen auf Dauer gleichmäßigen Sitz und eine gleichmäßige Ausrichtung des Ablenkelementes erzielen, selbst wenn ein solches Endoskop zahlreiche Reinigungszyklen durchschritten hat.

Nach Anlegen des Bruchstückes ist dieses fest und unverlierbar durch die Halterung gehalten. Diese Halterung dient eben nur zur Halterung des Bruchstückes und hat keinen Einfluss auf die Orientierung des Ablenkelements. Daher ist es beispielsweise möglich, diese Halterung als eine Schraubkappe auszubilden.

In einer weiteren Ausgestaltung der Erfindung ist das Bruchstück magnetisch ausgebildet und ist magnetisch am Körper des Endoskopkopfes haltbar.

Diese Maßnahme hat den Vorteil, dass das Zerlegen und das Montieren sehr erleichtert wird.

Beim Zerlegen kann man z.B. zunächst eine Halterung lösen, beispielsweise die zuvor erwähnte Schraubkappe abschrauben. Aufgrund der magnetischen Halterung ist sichergestellt, dass das Bruchstück nicht vom Körper des Endoskops abfällt. Dazu muss es von Hand oder mit einem Werkzeug ergriffen werden. Gleichermaßen ist dann nach einem Reinigungs- und Sterilisiervorgang die Montage wieder erleichtert. Durch die Magnetwirkung wird das Bruchstück nach Anlegen an den Körper des Endoskopkopfes gehalten bis es anschließend durch eine entsprechende Halterung unverrückbar gesichert ist.

Man kann das Bruchstück ansetzen, den korrekten Sitz überprüfen und dann beispielsweise durch Eindrücken eine Klipsverbindung schließen.

In einer weiteren Ausgestaltung der Erfindung weist das Bruchstück etwa die Form eines Kreisabschnittes auf, von dem das Ablenkelement vorspringt.

Diese Maßnahme hat unter anderem den Vorteil, dass bei diesem doch relativ kleinen Bauteil ein relativ stabiler, etwa scheibenabschnittförmiger Körper gebildet ist, der einfach beim Montieren an den entsprechenden Ausschnitt im Körper angesetzt werden kann. Von dem Körper steht dann das eigentliche Ablenkelement vor, bzw. der Körper selbst kann dann schon als ein Teil des Ablenkelementes ausgebildet sein.

In einer weiteren Ausgestaltung der Erfindung sind beim Vorsehen mehrerer Kanäle zum Führen von Spülmedien entsprechend mehrere Ablenkelemente am Bruchstück vorhanden.

Diese Maßnahme hat den Vorteil, dass, falls z.B. ein erster Kanal zum Zuführen eines flüssigen Mediums, z.B. Spülwasser, und ein zweiter Kanal zum Zuführen eines gasförmigen Mediums vorgesehen ist, der Lichteinlass zunächst mit dem flüssigen Spülmedium beaufschlagt werden kann. Dieser ist mit einem entsprechend über der Mündungsöffnung dieses Kanals angeordneten Ablenkelement versehen. Ferner ist an einem davon abseits gelegenen Kanal zur Zuführung von Spülluft ein entsprechendes weiteres Ablenkelement vorgesehen, um die Luft anschließend zum Feinspülen oder zum Trocknen über den Lichteinlass führen zu können. Es ist möglich, diese mehreren Ablenkelemente als ein integrales Ablenkelement an dem Bruchstück vorzusehen, so dass ein gewisser Bereich jeweils über einer Öffnung einer Mündung eines der beiden Spülkanäle zum Liegen kommt.

In einer weiteren Ausgestaltung der Erfindung ist im Bruchstück zumindest eine Öffnung für einen weiteren Kanal, insbesondere für einen Instrumentenkanal, ausgespart.

Diese Maßnahme hat den Vorteil, dass das Bruchstück einen relativ großen Bereich des Endes des Körpers des Endoskopkopfes darstellen kann, beispielsweise eine halbe Scheibe oder mehr, und dadurch dann relativ stabil ist. Dann ist es möglich, durch den Körper eine durchgehende Öffnung auszusparen, um beispielsweise einen in diesem Bereich liegenden Instrumentenkanal freizuhalten. Dies trägt zur Stabilität des Bruchstückes bei und erlaubt es, dieses als relativ großes Bauteil auszubilden, das dann entsprechend handhabbar ist.

In einer weiteren Ausgestaltung der Erfindung ist das Ablenkelement derart ausgebildet, dass es das Spülmedium nicht nur auf einen Lichteinlass sondern auch auf einen Beleuchtungslichtauslass oder gegebenenfalls mehrere Auslässe richtet.

Es ist zwar das Hauptbestreben, den Lichteinlass freizuspülen, um ein optimales Beobachtungsergebnis zu erzielen. Die Lichtausbeute ist aber manchmal dadurch verringert, dass die Lichtauslassöffnung des Beleuchtungslichtes durch Verschmutzungen abgedeckt bzw. dann entsprechend abgedunkelt ist. Durch die nun vorgeschlagene konstruktive Maßnahme kann nicht nur der Lichteinlass, sondern auch die Beleuchtungslichtauslässe freigespült bzw. freigeblasen werden.

In einer weiteren Ausgestaltung der Erfindung ist auf der distalen Stirnseite des Körpers zwischen einem Kanal zum Führen des Spülmediums und dem zu spülenden Einlass bzw. Auslass jeweils eine kanalartige Ausnehmung zum Führen des Spülmediums ausgespart.

Diese Maßnahme hat den Vorteil, dass dadurch eine zielgerechte Führung des Spülmediums, das aus der Mündung des Kanals austritt, zum zu spülenden Einlass bzw. Auslass durchgeführt wird. Das Ablenkelement, das über den kanalartigen Ausnehmungen zum Liegen kommt, stellt eine Art Deckel dieser schachtartigen bzw. kanalartigen Ausnehmung dar, so dass insgesamt ein Führungskanal für das Spülmedium geschaffen wird.

Je nach Ausgestaltung dieser kanalartigen Ausnehmungen wird das Spülmedium von einem Kanal bzw. dessen Mündungsöffnung gezielt zu einem oder mehreren Einlässen bzw. Auslässen für die lichtführenden Medien geführt.

Diese Maßnahme hat den Vorteil, dass eine besonders zielgerechte Führung der Spülmedien erzielt werden kann. Die kanalartigen Aussparungen sind bei der originären Herstellung des Körpers, der beispielsweise als Spritzgussteil hergestellt werden kann, entsprechend zu bewerkstelligen.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand eines ausgewählten Ausführungsbeispiels im Zusammenhang mit den beiliegenden Zeichnungen näher beschrieben und erläutert. Es zeigen:
- Fig. 1: ein Endoskop, nämlich ein flexibles Videoendoskop, das mit einem erfindungsgemäßen Endoskopkopf mit Ablenkelement versehen ist, wobei mittig eine etwas vergrößerte Draufsicht auf das distale Ende dargestellt ist,
- Fig. 2: eine Explosionsdarstellung der wesentlichen Bauelemente des distalen Endoskopkopfes,
- Fig. 3: den Endoskopkopf im montierten Zustand mit eingeschobener Optik,
- Fig. 4: einen Schnitt längs der Linie IV-IV von Fig. 2,
- Fig. 5: einen Schnitt längs der Linie V von Fig. 2, und
- Fig. 6: einen dem Schnitt von Fig. 4 entsprechenden Schnitt, bei dem das Bruchstück, wie es in Fig. 5 dargestellt ist, aufgesetzt ist.

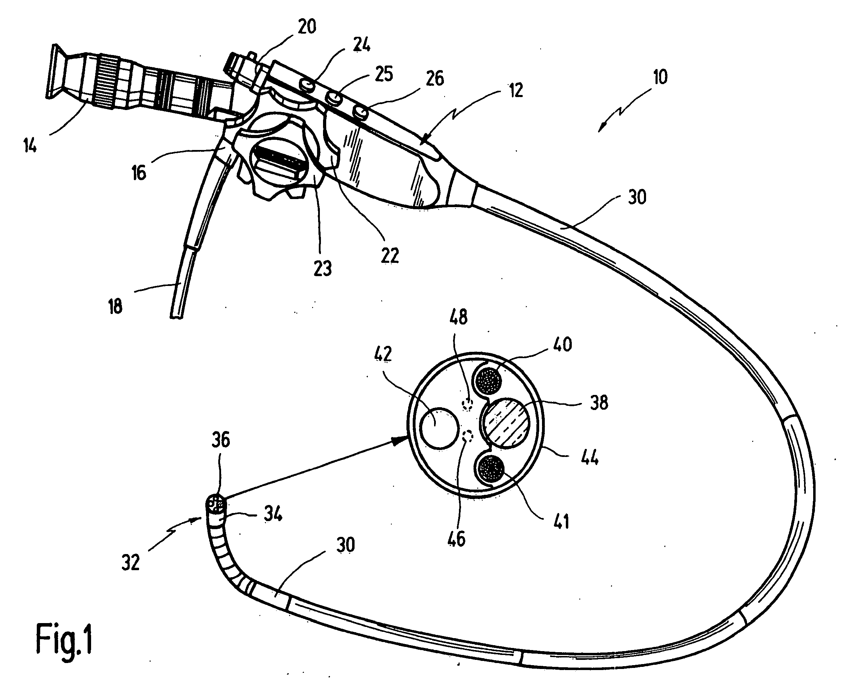

In Fig. 1 ist ein erfindungsgemäßes flexibles Endoskop in seiner Gesamtheit mit der Bezugsziffer 10 bezeichnet.

Das flexible Endoskop 10 weist proximal ein insgesamt mit der Bezugsziffer 12 bezeichnetes Kopfstück auf. Proximalseitig steht vom Kopfstück 12 ein Okular 14 vor. Ein seitlich vorstehender Anschluss 16 dient dazu, einen Leitungsstrang 18 anzuschließen, der Leitungen für Beleuchtung, Spülung, Insufflation, Absaugung, Bildleitung und dergleichen enthalten kann. Ein weiterer, sich etwa in Richtung des Okulars 14 erstreckender Anschluss 20 ist dazu vorgesehen, dass über diesen Instrumente, beispielsweise Zangen, Schlingen oder dergleichen, in das Endoskop 10 bzw. das Kopfstück 12 eingeschoben werden können.

Seitlich am Kopfstück 12 sind zwei Handräder 22, 23 angeordnet, mit denen die Blickrichtung des Endoskops verstellt werden kann. Im Bereich des Kopfstücks 12 sind ferner Schalter 24, 25, 26 angeordnet, über die verschiedene Funktionen, wie beispielsweise Saugen, Spülen oder dergleichen, gesteuert werden können, wie das an sich bei der Ausgestaltung von flexiblen Endoskopen bekannt ist.

Vom Kopfstück 12 streckt sich ein langerstreckter flexibler Schaft 30 fort, der im dargestellten Ausführungsbeispiel etwa die Länge von einem Meter aufweist. Der Schaft 30 ist aus einem flexiblen Kunststoffmaterial hergestellt, das ein Krümmen und Biegen des Schaftes 30 ermöglicht.

Endseitig ist der Schaft 30 über einen Endoskopkopf 32 abgeschlossen. Aus der vergrößerten distalen, stirnseitigen Endansicht ist zu entnehmen, dass dort ein Lichteinlass 38 mündet, über den Licht in das Innere des Schaftes 30 eintreten kann.

Dieses Licht entspricht dem Bild, das über das Okular 14 beobachtet werden kann. Vom Lichteinlass 38 wird, je nach Konstruktion des Endoskops, das Licht über Lichtleiter, sei es ein starres Linsensystem oder, bei flexiblem Endoskop, ein flexibler Lichtleiter, weitergeleitet. Bei der Ausgestaltung als Videoendoskop wird das eintretende Licht über einen Chip, einen so genannten CCD-Chip, in elektrisches Signal überführt und dann über den Leitungsstrang 18 einem Monitor oder einem Bildverarbeitungssystem zugeführt.

Ferner ist zu sehen, dass zwei Beleuchtungslichtauslässe 40, 41 vorhanden sind, über die Beleuchtungslicht dem distalen Ende zugeführt wird und aus diesem austritt.

Ferner ist zu erkennen, dass ein durchgehender Instrumentenkanal 42 vorgesehen ist.

Durch diesen Instrumentenkanal 42 kann ein durch den Anschluss 20 eingeschobenes Instrument am distalen Ende austreten.

Durch gestrichelte Linien sind zwei Kanäle 46 und 48 angedeutet, wobei der Kanal 46 ein Luftkanal zum Zuführen von Spülluft ist und der Kanal 48 als wasserführender Kanal ausgebildet ist, um Wasser als Spülflüssigkeit dem distalen Ende zuzuführen.

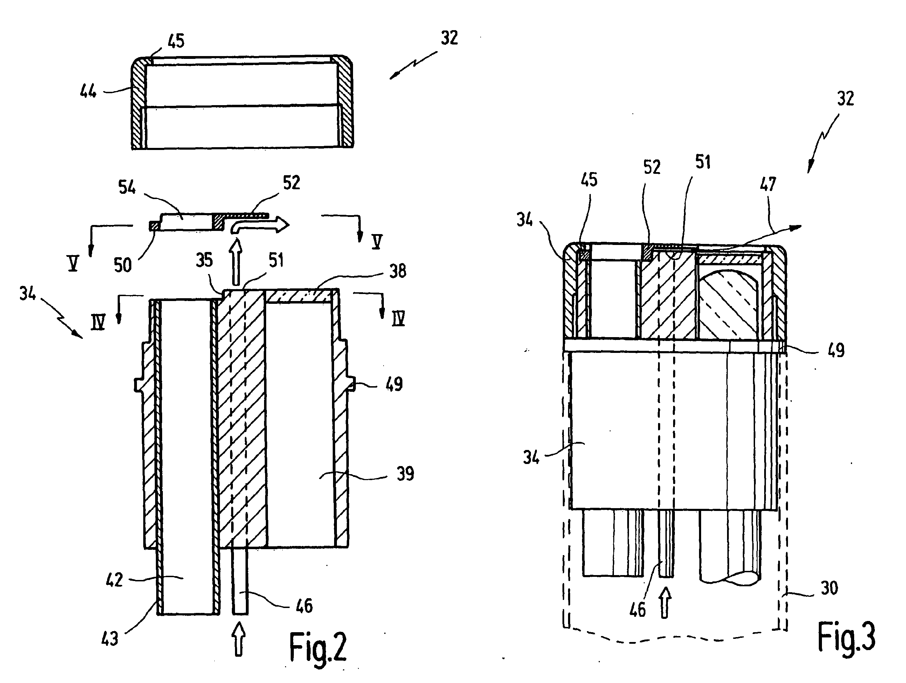

Wie aus Fig. 1 ersichtlich, liegen diese Kanäle 46 und 48 verdeckt unter einem Bauteil, das zunächst näher im Zusammenhang mit den Figuren 2 bis 6 beschrieben werden soll.

Aus der Explosionsdarstellung von Fig. 2 ist zu entnehmen, dass der Endoskopkopf 32 einen etwa zylindrischen Körper 34 aufweist.

Aus der Explosionsdarstellung ist ferner zu entnehmen, dass am distalen Endbereich des Körpers 34 ein Bruchstück 50 abgenommen werden kann, von dem seitlich ein Ablenkelement 52 vorspringt.

Das Bruchstück 50 wird, wenn es auf die Oberseite des Körpers 34 an die Lücke angelegt wird, durch Aufschrauben einer Ringkappe 44 gesichert, wie das aus Fig. 3 ersichtlich ist. Dazu weist die Ringkappe 44 einen entsprechenden Ringflansch 45 auf, der das Bruchstück 50 teilweise umfänglich etwas übergreift. Die Ringkappe 44 dient zur Halterung des Bruchstückes 50 am Körper 34.

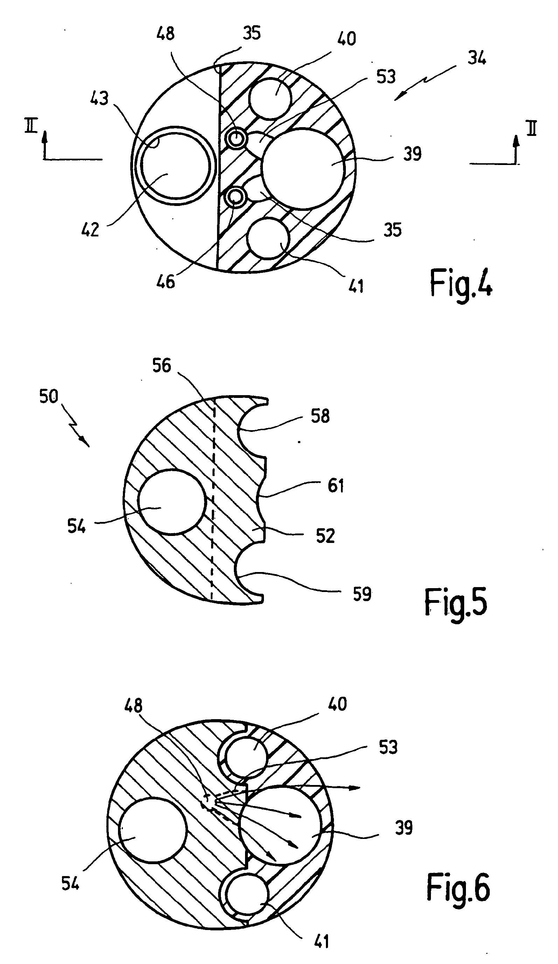

Aus der Schnittdarstellung von Fig. 5 im Zusammenhang mit der Darstellung von Fig. 2 ist zu erkennen, dass das Bruchstück 50 einen kreisabschnittförmigen Körper aufweist, der sich in der Darstellung von Fig. 5 etwa links von der gestrichelten Linie erstreckt. An der Oberseite des Körpers 34 ist eine entsprechende Stufe 35 vorhanden, an der dieser Abschnitt des Bruchstückes komplementär angelegt werden kann.

Damit der Instrumentenkanal 42 bis zum Ende durchreicht, ist eine entsprechende Öffnung 54 im Bruchstück 50 ausgespart.

Der Abschnitt, der sich in Fig. 5 rechts von der gestrichelten Linie 56 erstreckt, beinhaltet das eigentliche Ablenkelement 52. In diesem Bereich sind zwei halbrunde Ausnehmungen 58 und 59 ausgespart, sowie eine weitere gekrümmte Ausnehmung 61.

Ist das Bruchstück 50 auf das distale Ende des Körpers 34 aufgelegt, wie das beispielsweise in Fig. 6 und Fig. 3 ersichtlich ist, schmiegen sich die halbrunden Ausnehmungen 58, 59 und 61 um die Kontur der Beleuchtungslichtauslässe 40 und 51 sowie um den Lichteinlass bzw. um den Optikkanal 39 herum.

Aus Fig. 4 ist zu entnehmen, dass etwa mittig durch den Körper 34 die beiden Spülkanäle hindurchlaufen, nämlich der Luftkanal 46 und der Wasserkanal 48, wobei diese durch in den Körper 34, der selbst aus Kunststoff hergestellt ist, eingesetzte Metallröhrchen umgrenzt werden.

Wie aus der Schnittdarstellung von Fig. 2 bzw. Fig. 3 zu entnehmen ist, ist in den Instrumentenkanal 42 ein entsprechendes metallisches Rohrstück 43 eingesetzt.

Das Bruchstück 50 selbst ist entweder aus magnetischem Material hergestellt oder mit einem magnetischen Einsatz versehen, so dass es durch Magnetwirkung an der Oberseite des Körpers 34 haftet.

In diesem Zustand kommt die Unterseite des Ablenkelements 52 in einem geringen Abstand, etwa 2/10 mm, über der Mündungsöffnung 51 der Spülkanäle 46 und 48 zum Liegen. Wird nun beispielsweise durch den Kanal 46 Luft hindurchgeführt, wie das in Fig. 2 durch einen Pfeil dargestellt ist, trifft die aus der Mündung 51 dieses Kanals 46 austretende Luft an die Unterseite des Ablenkelements 52 und wird durch dieses seitlich in Richtung des Lichteinlasses 38 bzw. des Optikkanals 39 abgelenkt, wie das in Fig. 2 durch Pfeile, in Fig. 3 durch den Pfeil 47, bzw. in Fig. 6 durch die Strömungspfeile dargestellt ist.

Aus der Schnittdarstellung von Fig. 4 ist zu entnehmen, dass im Bereich zwischen dem Kanal 46 und dem Optikkanal 39 eine kanalartige Ausnehmung 55 an der Oberseite bzw. der Stirnseite des Körpers 34 vorhanden ist. Gleichermaßen ist zwischen dem Kanal 48 und dem Optikkanal 39 eine entsprechende kanalartige Ausnehmung 53 vorgesehen. Diese Ausnehmungen 53 und 55 dienen dazu, zielgerecht das aus den Kanälen 46 bzw. 48 austretende Spülmedium auf den Optikkanal 39 zu richten. Das Ablenkelement 52 deckt diese nach distal offenen Ausnehmungen 53 und 55 ab, wie das aus der Darstellung von Fig. 6 ersichtlich ist. Bei aufgesetztem Bruchstück 50 sind somit genau definierte Strömungs- bzw. Verbindungskanäle zwischen den Kanälen 46 und 48 zum Zuführen des Spülmediums in den zu spülenden Optikkanal 39 vorgesehen. In dieser Ausgestaltung kann beispielsweise der Optikkanal 39 abwechslungsweise und gezielt entweder mit Luft oder mit Wasser gespült werden.

Durch entsprechende andere Ausgestaltung bzw. Ausrichtung dieser Kanäle können auch die Beleuchtungslichtauslässe 40 und 41 gezielt gespült werden.

Wie insbesondere aus Fig. 6 zu entnehmen ist, erstreckt sich das Ablenkelement 52 so, dass beispielsweise aus dem Kanal 48 austretende Spülflüssigkeit nicht nur über den Optikkanal 39 sondern auch über den entsprechenden Beleuchtungslichtauslass 40 geführt wird, so dass dieser auch gleichzeitig von Verschmutzungen freigespült wird. Entsprechendes gilt dann für den Luftkanal 46.

Zum Reinigen bzw. Sterilisieren wird die Ringkappe 44 abgeschraubt, wie das beispielsweise aus der Explosionsdarstellung von Fig. 2 ersichtlich ist, aufgrund der Magnetwirkung bleibt das Bruchstück 50 zunächst aber an Ort und Stelle. Dies kann nunmehr abgenommen werden, so dass dann beispielsweise die Kanäle 46 und 48 mittels hindurchgeschobener Bürsten gereinigt werden können.

Nach dem Reinigen und Sterilisieren wird das Bruchstück 50 wieder an der Oberseite des Körpers 34 angesetzt, wobei dies dadurch erleichtert wird, dass dieses von oben und seitlich an die Kante 35 herangeschoben wird. Durch ein Aufdrehen der Ringkappe 44 wird dieses Teil dann exakt zentriert und fixiert. Dazu greift ein entsprechendes, hier nicht näher bezeichnetes Innengewinde der Ringkappe 44 in ein entsprechendes Außengewinde am Körper 34 ein.

Ein am Körper 34 umlaufender Ringflansch 49 begrenzt diese Bewegung. Auf der gegenüberliegenden Seite begrenzt dieser Ringflansch 49 die Einstecktiefe des Endoskopkopfes 32 in den Schaft 30.

## Patentansprüche

1. Endoskop mit einem Schaft (30), der distal in einem Endoskopkopf (32) endet, der zumindest einen Lichteinlass (38) und zumindest einen Kanal (46, 48) zum Durchführen eines Spülmediums aufweist, sowie mit einem Ablenkelement (52), das im Abstand vor einer Mündung (51) des Kanals (46, 48) angeordnet ist, um das austretende Spülmedium zum Lichteinlass (38) zu lenken, wobei das Ablenkelement (52) an einem Bruchstück (50) des Körpers (34) des Endoskopkopfes (32) angeordnet ist, das vom Körper (34) abnehmbar ist, und dass das Bruchstück (50) lagedefiniert und unverrückbar am Körper (34) aufgenommen und über eine Halterung am Körper (34) befestigbar ist, **dadurch gekennzeichnet, dass** die Halterung als Kappe (44) ausgebildet ist, die auf den Zusammenbau aus Bruchstück (50) und Endoskopkopf (32) aufsetzbar ist.

2. Endoskop nach Anspruch 1, **dadurch gekennzeichnet, dass** das Bruchstück (50) magnetisch ausgebildet ist und magnetisch am Körper (34) haltbar ist.

3. Endoskop nach Anspruch 1 oder 2, **dadurch gekennzeichnet, dass** das Bruchstück (50) etwa die Form eines Kreisabschnitts aufweist, von dem das Ablenkelement (52) vorspringt.

4. Endoskop nach einem der Ansprüche 1 bis 3, **dadurch gekennzeichnet, dass** beim Vorsehen mehrerer Kanäle (46, 48) zum Führen von Spülmedien entsprechend mehrere Ablenkelemente (52) am Bruchstück (50) vorhanden sind.

5. Endoskop nach einem der Ansprüche 1 bis 4, **dadurch gekennzeichnet, dass** im Bruchstück (50) zumindest eine Öffnung (54) für einen weiteren Kanal, insbesondere einen Instrumentenkanal (42), ausgespart ist.

6. Endoskop nach einem der Ansprüche 1 bis 5, **dadurch gekennzeichnet, dass** das Ablenkelement (52) derart ausgebildet ist, dass es das Spülmedium nicht nur auf einen Lichteinlass (38), sondern auch auf Beleuchtungslichtauslässe (40, 41) richtet.

7. Endoskop nach einem der Ansprüche 1 bis 6, **dadurch gekennzeichnet, dass** auf der distalen Stirnseite des Körpers (34) zwischen einem Kanal (46, 48) zum Führen eines Spülmediums und dem zu spülenden Einlass (38) bzw. Auslass (40, 41) kanalartige Ausnehmungen (53, 55) zum Führen des Spülmediums ausgespart sind.

## Claims

1. Endoscope with a shaft (30) which, at its distal ends, terminates in an endoscope head (32) having at least one light inlet (38) and at least one channel (46, 48) for passage of a flushing medium, and further with a deflecting element (52) which is arranged at a distance in front of a mouth (51) of the channel (46, 48) in order to guide the emerging flushing medium to the light inlet (38), said deflecting element (52) is arranged on a segment (50) of the body (34) of the endoscope head (32), said segment (50) being able to be detached from the body (34), and said segment (50) is received captively and in a defined position on the body (34) and is attachable to said body (34) via a holder, **characterized in that** the holder is designed as a cap (44) which can be put onto the assembly of segment (50) and endoscope head (32).

2. Endoscope of claim 1, **characterized in that** the segment (50) is magnetic and can be held magnetically on the body (34).

3. Endoscope of claims 1 or 2, **characterized in that** the segment (50) has approximately the shape of a portion of a circle from which the deflecting element (52) protrudes.

4. Endoscope of anyone of claims 1 through 3, **characterized in that**, when a plurality of channels (46, 48) are provided for passage of flushing media, a plurality of deflecting elements (52) are correspondingly present on the segment (50).

5. Endoscope of anyone of claims 1 through 4, **characterized in that** at least one opening (54) for a further channel, in particular an instrument channel (42), is cut out in the segment (50).

6. Endoscope of anyone of claims 1 through 5, **characterized in that** the deflecting element (52) is designed in such a way that it directs the flushing medium not only to a light inlet (38) but also to illumination light outlets (40, 41).

7. Endoscope of anyone of claims 1 through 6, **characterized in that** on the distal face of the body (34), between a channel (46, 48) for guiding a flushing medium and the inlet (38) or outlet (40, 41) to be flushed, channel-like recesses (53, 55) are cut out for guiding the flushing medium.

## Revendications

1. Endoscope comprenant une gaine (30) qui se termine distalement par une tête d'endoscope (32) qui présente au moins une entrée de lumière (38) et au moins un canal (46, 48) pour le passage d'un fluide de rinçage, ainsi que comprenant un élément déflecteur (52) qui est disposé à distance devant une embouchure (51) du canal (46, 48) pour diriger le fluide de rinçage sortant vers l'entrée de lumière (38), l'élément déflecteur (52) étant disposé sur une partie fractionaire (50) du corps (34) de la tête d'endoscope (32) qui peut être démontée du corps (34), la partie fractionaire (50) étant montée dans une position définie et de manière fixe sur le corps (34) et pouvant être fixée sur le corps (34) par un moyen de fixation, **caractérisé en ce que** le moyen de fixation est réalisé sous la forme d'un capuchon (44) qui peut être posé sur l'ensemble formé de la partie fractionaire (50) et de la tête d'endoscope (32).

2. Endoscope selon la revendication 1, **caractérisé en ce que** la partie fractionaire (50) est magnétique et peut être maintenue magnétiquement sur le corps (34).

3. Endoscope selon la revendication 1 ou 2, **caractérisé en ce que** la partie fractionaire (50) présente à peu près la forme d'un segment de cercle duquel l'élément déflecteur (52) fait saillie.

4. Endoscope selon l'une des revendications 1 à 3, **caractérisé en ce que** si plusieurs canaux (46, 48) sont prévus pour conduire des fluides de rinçage, une pluralité correspondante d'éléments déflecteurs (52) est présente sur la partie fractionaire (50).

5. Endoscope selon l'une des revendications 1 à 4, **caractérisé en ce qu'**au moins une ouverture (54) pour un autre canal, en particulier un canal d'instrument (42), est pratiquée dans la partie fractionaire (50).

6. Endoscope selon l'une des revendications 1 à 5, **caractérisé en ce que** l'élément déflecteur (52) est réalisé de façon à diriger le fluide de rinçage non seulement vers une entrée de lumière (38), mais aussi vers des sorties de lumière d'éclairage (40, 41).

7. Endoscope selon l'une des revendications 1 à 6, **caractérisé en ce que** des évidements (53, 55) sous forme de canal servant à conduire le fluide de rinçage sont pratiqués du côté frontal distal du corps (34) entre un canal (46, 48) servant à conduire un fluide de rinçage et l'entrée (38) ou la sortie (40, 41) à rincer.
